(19) Europäisches Patentamt
European Patent Office
Office européen des brevets

(11) Publication number: **0 225 997 A2**

(12) # EUROPEAN PATENT APPLICATION

(21) Application number: 86114701.5

(22) Date of filing: 23.10.86

(51) Int.Cl.[4]: A 61 K 31/17

(30) Priority: 24.10.85 EP 85113545

(43) Date of publication of application: 24.06.87 Bulletin 87/26

(84) Designated Contracting States: AT BE CH DE ES FR GB GR IT LI LU NL SE

(71) Applicant: Die Deutsches Primatenzentrum GmbH
Kellnerweg 4
D-3400 Göttingen(DE)

(72) Inventor: Jentsch, Klaus-Dieter, Dr.
DPZ, Kellnerweg 4
D-3400 Göttingen(DE)

(72) Inventor: Hunsmann, Gerhard, Prof. Dr.
DPZ, Kellnerweg 4
D-3400 Göttingen(DE)

(72) Inventor: Nickel, Peter, Prof. Dr.
Pharmazeutisches Institut der Universität Bonn
An der Immenburg 4 D-5300 Bonn 1(DE)

(74) Representative: Lederer, Franz, Dr. et al,
Vanderwerth, Lederer & Riederer Patentanwälte
Lucile-Grahn-Strasse 22
D-8000 München 80(DE)

(54) **Use of urea derivatives for preparing a pharmaceutical composition for the treatment of a virus-induced disease.**

(57) Use of urea derivatives of the formula:

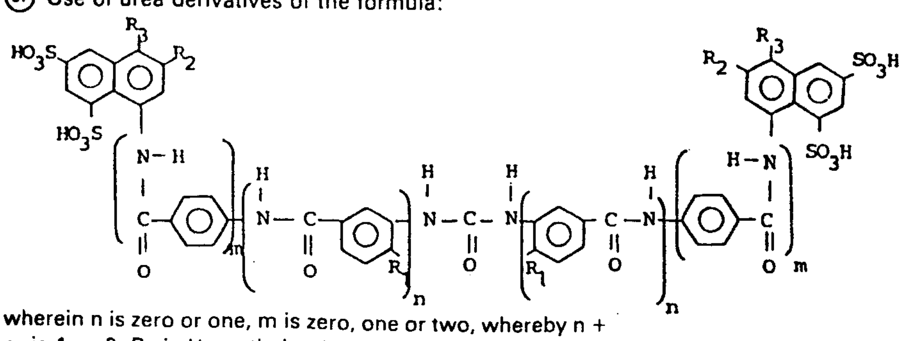

wherein n is zero or one, m is zero, one or two, whereby n + m is 1 or 2, $R_1$ is H, methyl, ethyl, propyl, isopropyl, butyl, isobutyl, phenyl or F, $R_2$ is H or $SO_3H$ and $R_3$ is H or $SO_3H$, with the proviso that $R_2$ and $R_3$ are not the same and pharmaceutically acceptable salts thereof for preparing a pharmaceutical composition for the treatment of a virus-induced disease.

EP 0 225 997 A2

Patentanwälte

(1934 - 1974)

DR. FRANZ LEDERER
Dipl.-Chem.

ANTON FREIHERR
RIEDERER v PAAR
Dipl.-Ing. Landshut

8000 MÜNCHEN 80
Lucile-Grahn-Straße 22
Telefon (089) 47 29 47
Telex 524 624 leder d
Telefax (089) 470 57 23 (Gr. II u. III)

0225997

TITLE MODIFIED
see front page

Die Deutsches Primatenzentrum GmbH
Kellnerweg 4
3400 Göttingen

23. Okt. 1986

# Use of Urea Derivatives for Preparing a Pharmaceutical Composition

The present invention relates to the use of urea derivatives for preparing a pharmaceutical composition useful for the treatment of a virus-induced disease.

Viruses and especially retroviruses cause a wide spectrum of diseases in animals and especially in man including various types of malignancies and non-malignant diseases such as immunodeficiency. Recently retroviruses designated as lymphadeno-pathy associated virus (LAV), human T-lymphotropic virus type III (HTLV III) or AIDS-related virus (ARV) have been isolated from patients with aquired Immunodeficiency Syndrome (AIDS) or AIDS related conditions. Seroepidemiological evidence, repeated isolation as well as retrospecitive examinations of accidental transmission of these viruses to man and experimental transmission of human isolates to certain primate species provide convincing evidence that these viruses are causitive agents of AIDS and related disorders in man. The exponentially raising number of patients suffering from a virus-induced disease, shows dramatically the urgent need for an effective pharmaceutical agent.

A protection against viral infection, replication and persistance may be achieved by different means.

Passive and active immunization can be one route. High titered immune sera against envelope polypeptides predominantly from murin and feline viruses have successfully been used to prevent leukemia and sarkoma in mice and cats. Moreover, purified viral polypeptides were efficient as vaccines for active immunisation. On the other hand some viruses show continuous alternation of the envelope polypeptides, so that the immune sera directed against one envelope polypeptide may not be active against another newly developed envelope polypeptide of the same virus.

Alternatively absorbtion and penetration of the viruses might be blocked by receptor binding compounds. Such a method, however, may cause some difficulties. Virus receptors and especially retrovirus receptors for human viruses have not been studied thoroughly and interference with these cell surface receptors is likely to disturb physiological functions of uninfected cells as well. In other words the side effects of these compounds can not be predicted.

Therefore a compound blocking a step in the life cycle of a virus, which is specific to the virus, would probably be very helpful in the treatment of a virus-induced disease. The life cycle of retroviruses includes the characteristic step of reverse transcription and integration of the DNA provirus into the genome of the host cell. This crucial step of infection is catalysed by the virus specific enzyme, reverse transcriptase (RT). This enzyme comprises three distinguishable activities, namely RNA dependant DNA polymerase and DNA polymerase, an RNAse (RNAseH) and an endonuclease activity. Compounds

inhibiting the reverse transcriptase may be active against a wide spectrum of retroviruses and should not interfere with the metabolism of uninfected cells. If the infection of new target cells plays an important role in the ethiology of a retrovirus induced disease, the compounds inhibiting the reverse transciptase can be very effective for prevention and treatment of retrovirus-induced diseases.

Recently suramin and other compounds whose RT inhibiting activity were known (De Clerq, Cancer Letters, 8 page 9-22, 1979), have been shown to reduce HTLV-III replications in culture (Mitsuya et al. Science, 226 page 172-174, 1984). One advantage of suramin is that it is a licensed drug, a disadvantage, however, is that large amounts of this substance have to be administered, which often lead to serious complications due to the systemic toxicity of this compound. Compounds which are more effective and less toxic than suramin are therefore highly desirable.

The present invention relates to urea derivatives of the formula:

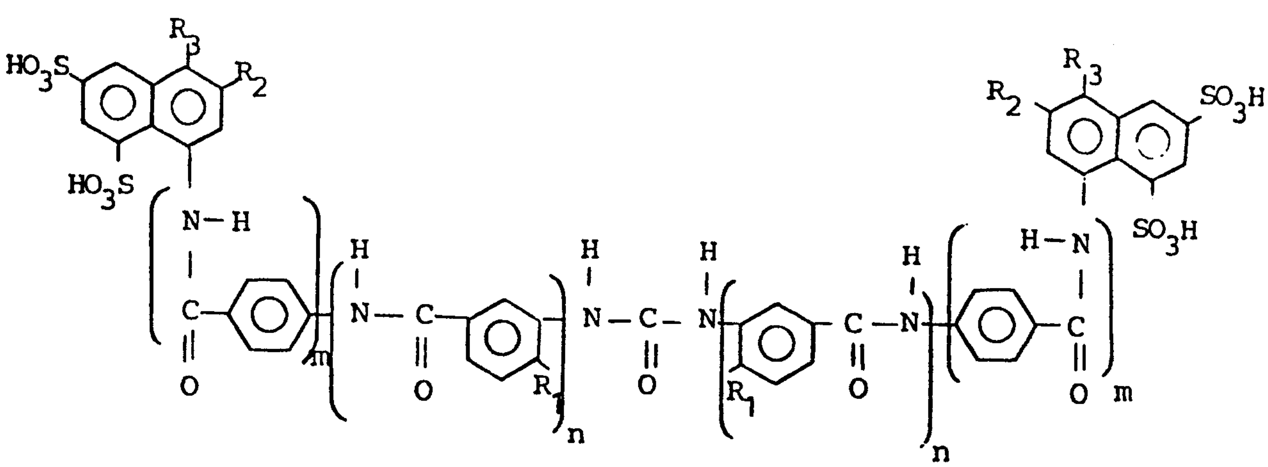

(I)

wherein n is zero or one, m is zero, one or two, whereby n + m is one or two, $R_1$ is H, methyl, ethyl, propyl, isopropyl, butyl, isobutyl, phenyl or F, $R_2$ is H or $SO_3H$ and $R_3$ is H or $SO_3H$ with the proviso that $R_2$ and $R_3$ are not the same and salts of the sulphonic acid groups with pharmaceutically acceptable bases and salts of the nitrogen groups with pharmaceutically acceptable acids, which are useful for the treatment of a virus-induced disease.

An additional interaction of the compounds according to the invention with some parts of the host cells, e.g. receptors, can cause a therapeutic effect not only in virus-induced diseases but also in diseases which may have any relation to viruses. Some types of cancer for example are induced by viruses and afterwards the viruses are not detectable in the cells. Therefore the compounds according to the invention can be advantageously administered to such diseases.

Especially useful for the treatment of a virus-induced disease are the compounds of the following formulae (II-VI):

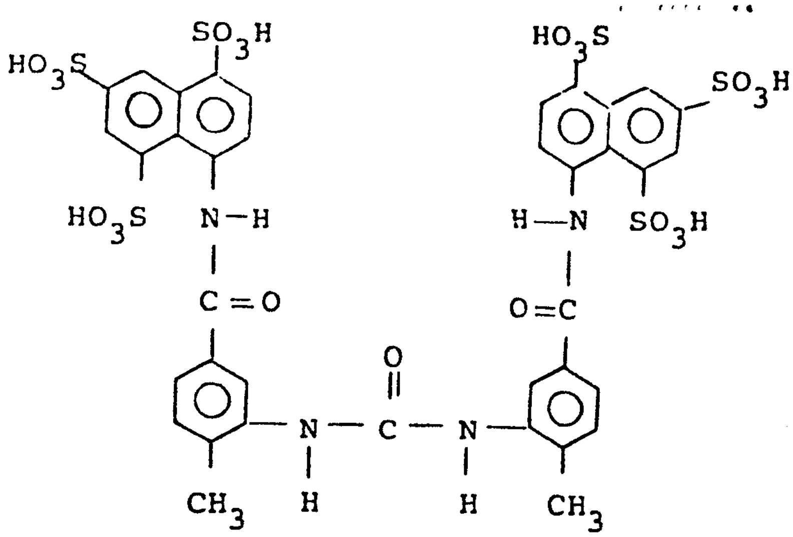

(II)

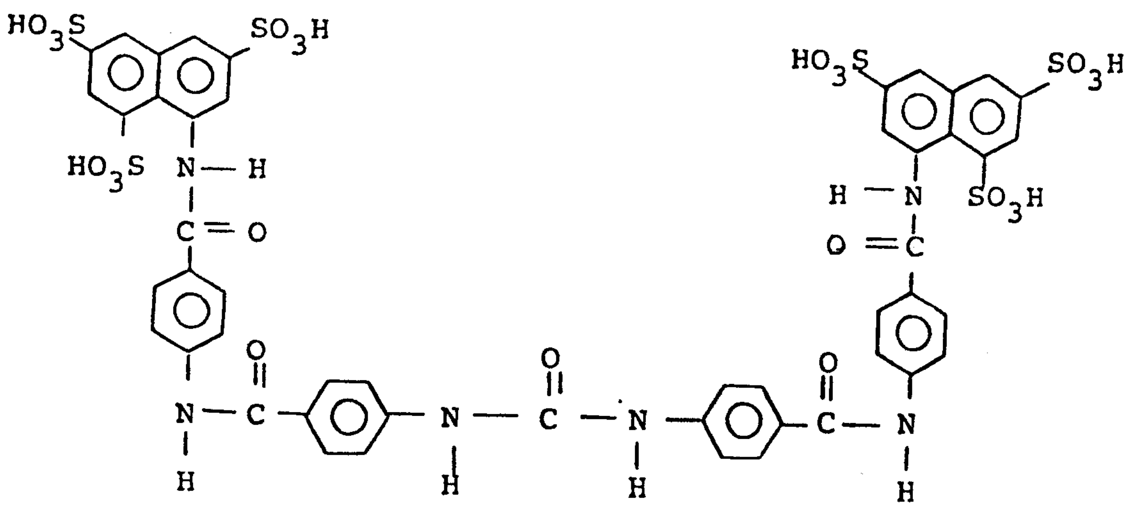

(III)

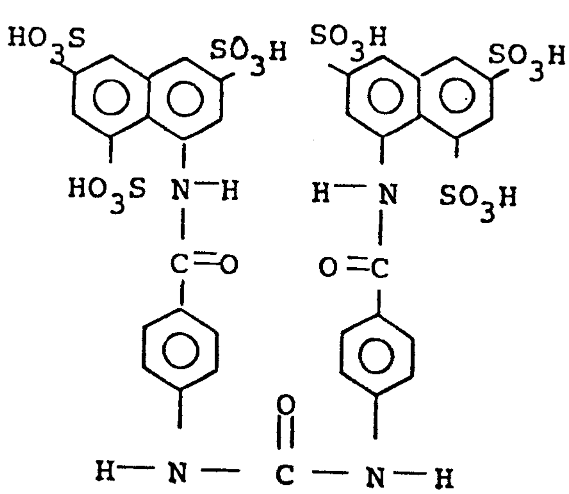

(IV)

0225997

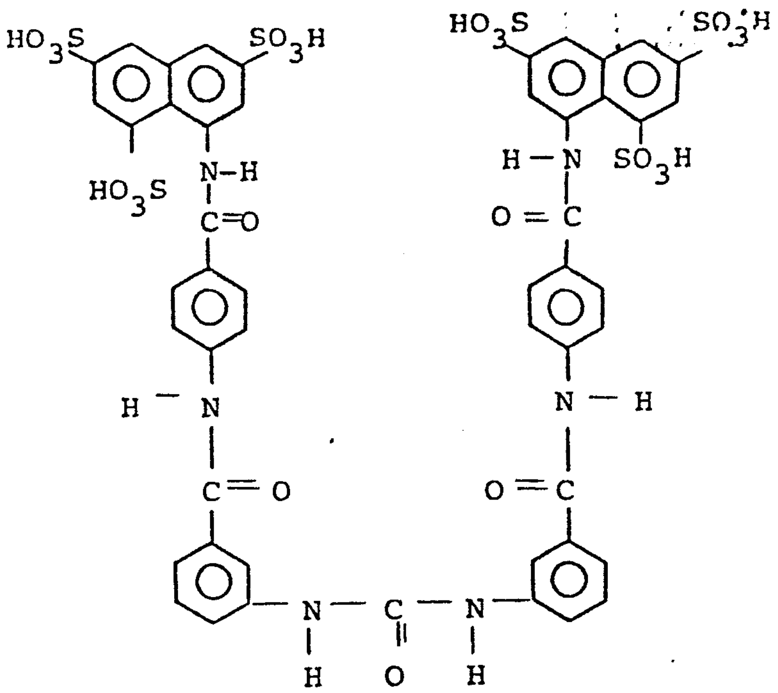

(V)

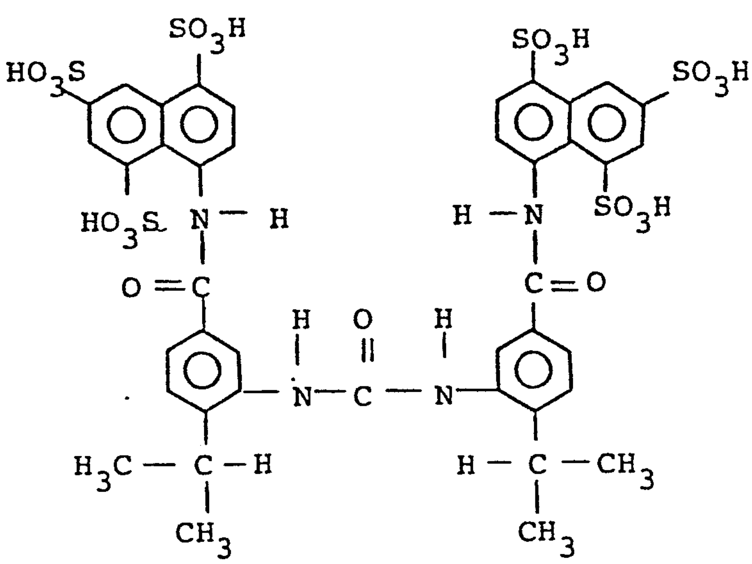

(VI)

The compounds according to the invention are more effective than suramin with respect to the inhibitory potency against the activity of the reverse transcriptase. Therefore smaller doses can be administered which lead to less toxic side effects. The synthetic routes to the compounds according to the invention are known to those skilled in the art and the synthesis of compounds according to the invention is examplary depicted in scheme I.

Scheme I

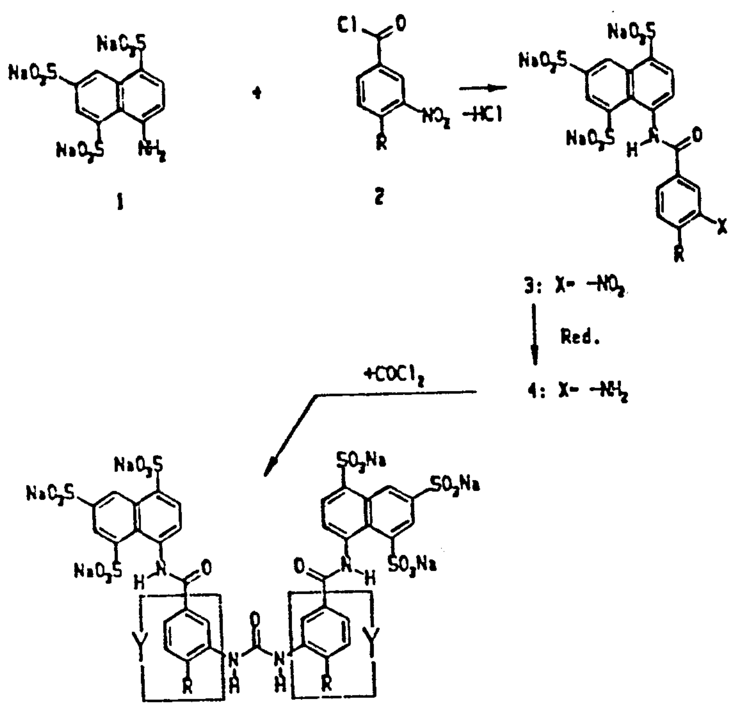

When used as antiviral agents in humans, the compounds of this invention are conveniently administered via the oral, subcutaneous, intramuscular, intravenous or intraperitoneal routes, generally in composition form. Such compositions include a pharmaceutical carrier selected on the basis of the chosen route of administration and standard pharmaceutical practice. For example, they can be administered in the form of tablets, pills, powders or granules containing such excipients as starch, milk sugar, certain types of clay, etc. They can be administered in capsules, in admixtures with the same or equivalent excipients. They can also be administered in the form of oral suspensions, solutions, emulsions, syrups and elixirs which my contain flavoring and coloring agents.

The physician will determine the dosage which will be most suitable for an individual patient and it will vary with the age, weight and response of the particular patient and the route of administration.

For parenteral administration, solutions of these urea derivatives in sesame or peanut oil or in propylene glycol or aqueous ethanol may be employed, as well as sterile aqueous solutions in distilled water. The aqueous solutions can be suitably buffered (pH 8) and the liquid diluent first rendered isotonic. These aqueous solutions are suitable for intravenous or intrathecal injection purposes. The oily solutions are suitable for intramuscular and subcutaneous injection purposes.

One suitable regimen is to administer a total dose of 3,1 g, a test dose of 100 mg. on day 0, followed by 500 mg. doses on days 3, 7, 14, 21, 28 and 35, each dose being given intervenously over 20 min.

0225997

Example 1.

Generally, the analysis of the urea derivatives of the present invention by conventional methods such as mass spectrometrical methods is restricted, because the molecules are involatile and thermolabile. Thus, under electron impact or chemical ionization only mixtures of thermal induced degradation products are detected. Therefore Fast Atom Bombardment (FAB), which in the negative mode is known as an excellent ionization method for sulphonated compounds was used. The urea derivatives were investigated by (-) FAB ionization using glycerol as the matrix. The compounds were easily solvated in the matrix due to the trisulfonated linkages of the molecules to give excellent abundances of the parent ions, detected as the molecular anions and Na salts. The (-) FAB spectra of the substituted urea analogs are shown in table 1 neglecting ions of the matrix, isotopical distributions and fragment ions of lower intensities. The compounds were investigated under the following general conditions:

The FAB ionizations in the negative mode were performed using a neutral atom bombardment gun (Ion tech, modified by AMD Intectra, Klein-Köhren 36, D-2833 Beckeln, BRD): the bombardment gas (Xe) was ionized and accelerated at 6 kV at 0,6 mA emission and 4 x $10^{-5}$ torr and adjusted on the target (glycerol matrix) in a distance lower than 10 mm. The ions obtained were accelerated and focussed at 3 kV in a conventional EI source (VARIAN MAT, CH 5 DF) at the lowest possible temperature to prevent thermal decompositions. High abundances of negative ions were obtained by the specially designed adjustable conversion dynode, which allows the reacceleration and refocussing of the converted ions (7 kV). For the registration a conventional secondary electron muliplier and photosensitive paper were used.

0225997

The mass scale was calibrated using CsJ/RbJ/NaJ cluster ions. The analysis of metastable decompositions was performed in the 2nd f.f.r. (DADI method) by focussing the parent ion at constant acceleration and magnetic field and by scanning the electric sector voltage. Compounds up to m/z 1800 were analyzed by reducing the accelerating voltage of the ion source to 2 kV.

Table 1: Parent and fragment ions of urea compounds in the (-)-FAB spectra (rel.intens. % B).

| Y | 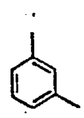 | 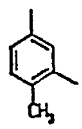 | 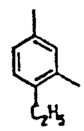 | 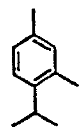 | 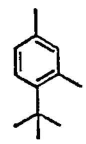 | 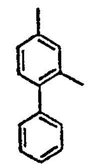 | 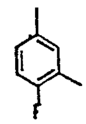 |
|---|---|---|---|---|---|---|---|
| X | 48 | 28 | 100 | 77 | 82 | 71 | 24 |
| NH | 90 | 67 | 61 | 92 | 82 | 91 | 68 |
| CO | 38 | 64 | 74 | 100 | 100 | 71 | 100 |
| Y | 25 | 11 | 9 | 23 | 55 | 9 | -- |
| NH | 40 | 25 | 9 | 48 | - | 71 | 32 |
| CO | 45 | 45 | 23 | 81 | 67 | 66 | 76 |
| NH | 47 | 39 | 43 | 75 | 11 | 100 | 48 |
| Y | 20 | 6 | - | 23 | - | 14 | 19 |
| CO | 12 | 3 | - | - | - | - | 11 |
| X | 100 | 100 | 33 | 44 | 22 | 37 | 84 |

X= 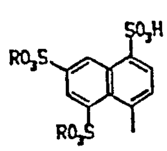 ( R=H,Na )

The consecutive fragmentations of the parent ions were investigated by MS/MS methods:
These methods allow the distinct analysis of the fragmentation processes of ions present in complex mixtures. The gun life time of the parent ions and their relatively high abundances enable the detection of the consecutive fragmentations, demonstrating the preferential cleavage of -NH-CO-bonds. Table 2 shows the fragmentation pattern of a model compound.

Table 2: Metastable Spectra of the urea compound ($C_{37}H_{25}N_4O_{21}S_6Na_4$; m/z: 1145) $Na_2$ -parent anion; the intensities are relative to all fragment anions (rel.% ).

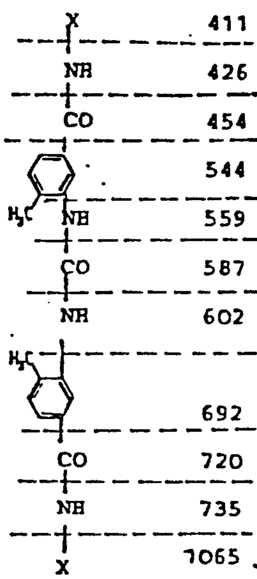

| m/z | rel.% |
|---|---|
| 411 | 2,6 |
| 426 | 19,1 |
| 454 | 2,6 |
| 544 | 1,5 |
| 559 | 12,8 |
| 587 | 16,3 |
| 602 | 8,1 |
| 692 | 1,3 |
| 720 | 15,3 |
| 735 | 2,6 |
| 1065 | 11,5 |
| 1128 | 6,4 |

X= 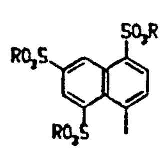 ( R=H,Na )

0225097

Complete fragmentations of the parent anions are detected of some compounds according to the invention (table 3):

Table 3: (-)-FAB spectra of urea derivatives the elemental composition and molecular weights of the alkali free acids and the $Na_6$-salts, the m/z values and relative intensities of the parent and fragment ions are given; the isotopic distributions and fragment ions of lower intensities are neglected.

X = naphthalene-1,3,5-trisulfonic acid

Y = substituted phenylene moiety with substituent R.

Acid: alkali free anions; $Na_x$ : alkali containing anions.

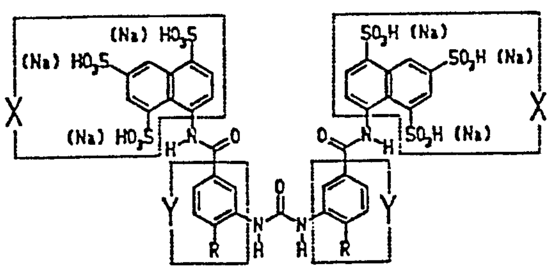

(R = H) $C_{35}H_{20}N_4O_{21}S_6Na_6$ ; MZ: 1162     Y = $C_6H_4$ , 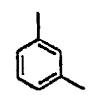

$C_{35}H_{26}N_4O_{21}S_6$ ; MZ: 1030

| Sequence | Acid | $Na_1$ | $Na_2$ | $Na_3$ |
|---|---|---|---|---|
| X | 367 (13) | 389 (13) | 411 (36) | -- |
| NH | -- | 404 (26) | 426 (91) | -- |
| CO | 410 (36) | -- | 454 (13) | -- |
| Y | 486 (13) | -- | 530 (20) | -- |
| NH | 501 (7) | -- | 545 (46) | -- |
| CO | 529 (20) | -- | 573 (39) | -- |
| NH | 544 (46) | -- | 588 (16) | -- |
| Y | 620 (13) | -- | 664 (13) | -- |
| CO | 648 (7) | -- | 692 (9) | -- |
| NH | 663 (13) | 685 (100) | 707 (13) | -- |
| X | 1029 (26) | 1051 (9) | 1073 (10) | 1095 (18) |

parent ions: m/z: 1117 (46) ($Na_4$) ; 1139 (13) ($Na_5$); 1161 (9) ($Na_6$)

other fragment ions: m/z: 350 (367-OH) (52); 324 (426-$SO_3$Na) (65).

Table 3 continued

Y = 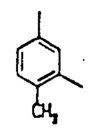 R = $CH_3$

$C_{37}H_{24}N_4O_{21}S_6Na_6$ ( MZ : 1190) ; $C_{37}H_{30}N_4O_{21}S_6$ ( MZ : 1058)

| Sequence | Acid | $Na_2$ |
|---|---|---|
| X | — | 411 (28) |
| NH | 382 (25) | 426 (42) |
| CO | 410(28) | 454 (36) |
| Y | — | 544 (11) |
| NH | — | 559(25) |
| CO | 543 (11) | 587 (33) |
| NH | 558 (25) | 602 (14) |
| Y | — | 692 (6) |
| CO | — | 720 (3) |
| NH | 691 (6) | 735 (6) |
| X | 1051 (22) | 1101 (7) |

parent and fragment ions: m/z(rel.intens.%B): 1189 (6; $Na_6$); 1167 (8; $Na_5$); 1145 (39; $Na_4$); 1123 (8; $Na_3$); 1079 (8; $Na_1$); 350(47; 367–OH); 324 (100; 426– $SO_3Na$); 302 (83; 404 – $SO_3Na$).

Table 3 , continued

Y = 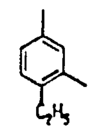 , R = $C_2H_5$

$C_{39}H_{28}N_4O_{21}S_6Na_6$ ( MZ : 1218) ; $C_{39}H_{34}N_4O_{21}S_6$ ( MZ : 1086)

| Sequence | Acid | $Na_2$ |
|---|---|---|
| X | 367 (27) | 411 (28) |
| NH | — | 426 (33) |
| CO | 410(28) | 454 (14) |
| Y | 514 (6) | — |
| NH | 529 (6) | — |
| CO | 557 (14) | — |
| NH | 572 (17) | 616 (8) |
| Y | — | — |
| CO | — | — |
| NH | — | — |
| X | 1085 (6) | — |

Parent and fragment ions: M/z(rel.intens.%B): 1173 (8; $Na_4$); 1005 (3); 719 (3; 1085– X); 350(100; 367 – OH).

Table 3, continued

Y = 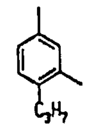 R = iso-propyl ($C_3H_7$)

$C_{41}H_{32}N_4O_{21}S_6Na_6$ ( MZ : 1246) ; $C_{41}H_{38}N_4O_{21}S_6$ ( MZ : 1114)

| Sequence | Acid | $Na_2$ |
|---|---|---|
| X | 367 (20) | 411 (33) |
| NH | 382 (27) | 426 (37) |
| CO | 410 (33) | 454 (36) |
| Y | — | 572 (16) |
| NH | — | 587 (36) |
| CO | 571 (16) | 615 (40) |
| NH | 586 (36) | 630 (16) |
| Y | 704 (9) | 748 (7) |
| CO | — | — |
| NH | 747 (7) | — |
| X | 1113 (13) | — |

Parent and fragment ions: m/z (rel.intens.%B): 1223 (20; $Na_5$); 535 (20; 615-$SO_3$); 485 (43; 587- $SO_3Na$); 469 (27; 572- $SO_3Na$); 513 (40; 615- $SO_3Na$); 383 (27; 485- $SO_3Na$); 367 (20; 469- $SO_3Na$); 350 (100; 367- OH); 324 (47; 426- $SO_3Na$); 309 (23; 411- $SO_3Na$); 248 (67).

Table 3, continued :

Y = 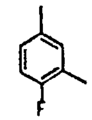 , R = F

$C_{35}H_{18}F_2N_4O_{21}S_6Na_6$ ( MZ : 1198) ; $C_{35}H_{24}F_2N_4O_{21}S_6$ ( MZ : 1066)

| Sequence | $Na_3$ |
|---|---|
| X | 433 (22) |
| NH | 448 (63) |
| CO | 476 (93) |
| Y | — |
| NH | 585 (29) |
| CO | 613 (71) |
| NH | 628 (44) |
| Y | 722 (18) |
| CO | 750 (10) |
| NH | 765 (10) |
| X | 1131 (7) |

Parent and fragment ions : m/z (rel.intens.%B): 1197 (15; $Na_6$); 1175 (10; $Na_5$); 1153 (27 ; $Na_4$); 1109 (6 ; [illegible]); 1087 (6 ; $Na_1$); 1051 (18; 1153- $SO_3Na$); 949 (11 ; 1051- $SO_3Na$); 511 (18 ; 613 -$SO_3Na$); 374 ( 18 ; 476 - $SO_3Na$); 350 ( 29 ; 367- OH); 346 ( 100 ; 448 - $SO_3Na$).

0225997

Table 3 continued

: Y = 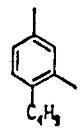 R = $C_4H_9$ (t-Butyl)

$C_{43}H_{36}N_4O_{21}S_6Na_6$ (MZ : 1274) ; $C_{43}H_{42}N_4O_{21}S_6$ ( MZ : 1142)

| Sequence | Acid | $Na_2$ |
|---|---|---|
| X | 367 (13) | 411 (17) |
| NH | 382 (13) | 426 (17) |
| CO | 410 (17) | 454 (20) |
| Y | — | 586 (20) |
| NH | — | — |
| CO | 585 (20) | 629 (5) |
| NH | — | 644 (4) |
| Y | — | — |
| CO | — | — |
| NH | — | — |
| X | — | — |

Parent and fragment ions: m/z (rel.intens.%B): 1229 (8 ; $Na_4$); 1114 (10; 1229-2 x $C_4H_9$); 1012 (5 ; 1114 - $SO_3Na$); 612 (30; 629- OH); 582 (7; 612- $SO_3Na$); 510 (33; 612- $SO_3Na$); 484 (27 ; 586- $SO_3Na$); 408 ( 17 ; 510- $SO_3Na$); 350 ( 100 ; 367- OH); 324 ( 47 ; 426- $SO_3Na$); 309 (27; 411- $SO_3Na$).

Table 3 continued

Y = 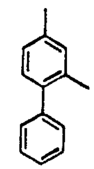 R = $C_6H_5$ (Phenyl)

$C_{47}H_{28}N_4O_{21}S_6Na_6$ ( MZ : 1314) ; $C_{47}H_{34}N_4O_{21}S_6$ ( MZ : 1182)

| Sequence | Acid | $Na_2$ |
|---|---|---|
| X | 367 (14) | 411 (21) |
| NH | 382 (17) | 426 (29) |
| CO | 410 (21) | 454 (14) |
| Y | — | 606 (4) |
| NH | — | 621 (36) |
| CO | 605 (4) | 649 (29) |
| NH | 620 (36) | 664 (14) |
| Y | — | 816 (7) |
| CO | — | — |
| NH | — | — |
| X | — | — |

Parent and fragment ions : m/z (rel.intens.%B): 1291 (19; $Na_5$); 1189 (10; 1291 - $SO_3Na$); 1087 (4; 1189- $SO_3Na$); 350 (100; 367- $SO_3Na$); 302 (21; 382- $SO_3$).

Table 4 FAB-Spectra

| | Compound acid/Na-salt | MZ | m/z (rel. intensities), detected signals of the intact molecule | $Na_1$ | $Na_2$ | $Na_3$ | $Na_4$ | $Na_5$ | $Na_6$ |
|---|---|---|---|---|---|---|---|---|---|
| formula IV | $C_{35}H_{26}N_4O_{21}S_6$ | 1030 | 1029 (25); | 1051 (8); | 1073 (9); | 1095 (15); | 1117 (40); | 1139 (11); | 1161 (8) |
| | $C_{35}H_{20}N_4O_{21}S_6Na_6$ | 1162 | | | | | | | |
| formula III | $C_{49}H_{36}N_6O_{23}S_6$ | 1268 | 1267 (2); | 1289 (4); | 1311 (3); | 1333 (3); | 1355 (4); | 1377 (3); | 1399 (4) |
| | $C_{49}H_{30}N_6O_{23}S_6Na_6$ | 1400 | | | | | | | |
| formula V | $C_{49}H_{36}N_6O_{23}S_6$ | 1268 | 1267 (3); | 1289 (5); | 1311 (4); | 1333 (3); | 1355 (2); | 1377 (2); | 1399 (3) |
| | $C_{49}H_{30}N_6O_{23}S_6Na_6$ | 1400 | | | | | | | |
| formula II | $C_{37}H_{30}N_4O_{21}S_6$ | 1058 | 1051 (22); | 1079 (8); | 1101 (7); | 1123 (8); | 1145 (39); | 1167 (8); | 1189 (6) |
| | $C_{37}H_{24}N_4O_{21}S_6Na_6$ | 1190 | | | | | | | |
| formula VI | $C_{41}H_{38}N_4O_{21}S_6$ | 1114 | 1113 (13); | | | | | 1223 (20) | |
| | $C_{41}H_{32}N_4O_{21}S_6Na_6$ | 1246 | | | | | | | |

Table 4 shows the FAB-spectra of the preferred compounds according to the invention.

Example 2

Reverse transcriptase assay:
RT assays were done according to standard procedures (Novak et al. J. of Virology, 1979, p. 438-452 and Poiesz et al. Proc. Natl. Acad. Sci. USA, 77, 1980, p. 7415-7419) with slight modifications. The final concentration was adjusted to 0.6% for Triton X-100 and 4 mM for $Mg^{2+}$. As template primer poly C. oligo dG. (Boehringer Mannheim) was added to 0.03 µg per 50 µl of reaction mixture. $^{32}P$-dGTP was obtained from Amersham Buchler (specific activity 3000 Ci/mmol). The specific activity in the reaction mixture was about 1 x $10^6$ cpm/pmole dGTP. To test the purity of the viral preparation poly A. oligo dT and poly dA. oligo dT were used as primer template. According to these tests the amount of contaminating cellular polymerases was less than 15% of total polymerase activity. With poly C. oligo dG no incorporation of radioactivity which would have resulted from cellular polymerase could be detected. The RT reaction was run at 42°C for 60 min. Reactions were stopped with trichloro acetic acid (TCA), sodiumpyrophosphate and salmsperm DNA followed by extensive washing of precipitates with 1 M HCl and 0,1 M sodiumpyrophosphate on Whatman GF/C filters. The amount of virus per sample was normalized to 25 µg/50µl protein concentration. Concentrations of suramin derivatives ranged from 40 µg to up to 1000 µg/ml. The dose response curve was plotted for each inhibitor and its 50% inhibitory dose ($ID_{50}$) was extrapolated from the curve.

| Compound | $ID_{50}$ (µg/ml) |
|---|---|
| formula II | 55 |
| formula III | 55 |
| formula IV | 60 |
| formula V | 80 |
| formula VI | 80 |
| Suramin | 90 |

Example 3

An aqueous propylene glycol solution containing the compound of formula II is prepared by dissolving the latter compound in propylene glycol-water (1:4 by weight) containing 1% by weight of trisodium phosphate and adjusted to an apparent pH of 8.0. The amount of compound employed is such that the resulting solution contains 50 mg. of the active ingredient per each ml. of solution. The solution is then sterilized by means of filtration through a 0.2 µm pore size cellulose membrane. The sterile aqueous propylene glycol solution so obtained is then suitable for intramuscular administration to humans.

Example 4

500 mg. of the compound of formula III are placed into a vial, sealed and then sterilized. For purposes of intravenous administration, a sufficient amount of distilled sterile water is added to each of the filled vials before use so as to ultimately provide a solution which contains 50 mg. of the active ingredient per each ml. of injectable solution.

## Claims

1. Use of urea derivatives of the formula:

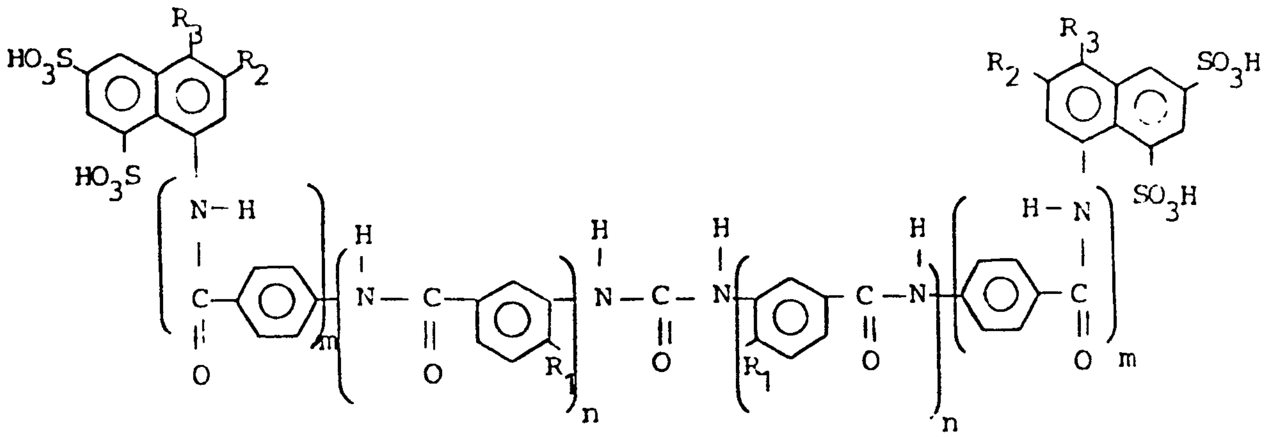

wherein n is zero or one, m is zero, one or two, whereby n + m is 1 or 2, $R_1$ is H, methyl, ethyl, propyl, isopropyl, butyl, isobutyl, phenyl or F, $R_2$ is H or $SO_3H$ and $R_3$ is H or $SO_3H$, with the proviso that $R_2$ and $R_3$ are not the same, and salts of the sulfonic acid groups with pharmaceutically acceptable bases and salts of the nitrogen groups with pharmaceutically acceptable acids for preparing a pharmaceutical composition for the treatment of a virus-induced disease.

2. Use of a urea derivative according to claim 1, wherein n is 1, $R_1$ is $CH_3$, m is zero, $R_2$ is H and $R_3$ is $SO_3H$.

3. Use of a urea derivative according to claim 1, wherein n is zero, m is 2, $R_2$ is $SO_3H$ and $R_3$ is H.

4. Use of a urea derivative according to claim 1, wherein n is zero, m is 1, $R_2$ is $SO_3H$ and $R_3$ is H.

5. Use of a urea derivative according to claim 1, wherein n is 1, $R_1$ is H, m is 1, $R_2$ is $SO_3H$ and $R_3$ is H.

6. Use of a urea derivative according to claim 1, wherein n is 1, $R_1$ is isopropyl, m is zero, $R_2$ is H and $R_3$ is $SO_3H$.

7. Use of urea derivatives according to one of the claims 1 to 6 for preparing a pharmaceutical composition for the treatment of a retrovirus-induced disease.

8. Use of urea derivatives according to one of the claims 1 to 6 for preparing a pharmaceutical composition for the treatment of a HTLV-III-induced disease.